(19) Europäisches Patentamt
European Patent Office
Office européen des brevets

(11) **EP 4 113 431 A1**

(12) **EUROPÄISCHE PATENTANMELDUNG**

(43) Veröffentlichungstag:
**04.01.2023  Patentblatt 2023/01**

(51) Internationale Patentklassifikation (IPC):
**G06T 5/50** *(2006.01)*   **A61B 6/00** *(2006.01)*

(21) Anmeldenummer: **21182696.1**

(22) Anmeldetag: **30.06.2021**

(52) Gemeinsame Patentklassifikation (CPC):
**G06T 5/50;** A61B 6/032; A61B 6/4241; A61B 6/481;
A61B 6/482; A61B 6/5258; G06T 2207/10081;
G06T 2207/20221; G06T 2207/30004

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH**
**91052 Erlangen (DE)**

(72) Erfinder:
• **Haag, Nicole**
  **91301 Forchheim (DE)**
• **Stierstorfer, Karl**
  **91052 Erlangen (DE)**

(54) **VERFAHREN UND VORRICHTUNG ZUR ERMITTLUNG EINES KORRIGIERTEN RÖNTGENBILDDATENSATZES**

(57)   Die Erfindung betrifft ein Verfahren zur Ermittlung eines korrigierten Röntgenbilddatensatzes umfassend die Schritte

- Bereitstellen (S1) eines vorläufigen spektralen Röntgenbilddatensatzes darstellend eine ortsaufgelöste Abschwächung von Röntgenstrahlung beim Durchgang durch ein Objekt (39) umfassend zumindest erste Bildwerte in Abhängigkeit eines ersten Röntgenenergieschwellwerts und zweite Bildwerte in Abhängigkeit eines zweiten Röntgenenergieschwellwerts,

- Korrigieren (S2) zumindest eines der ersten Bildwerte oder eines der zweiten Bildwerte des vorläufigen Röntgenbilddatensatzes mittels einer Skalierungsfunktion in Abhängigkeit eines Röntgenphotonenflusses und einer Kombination aus dem zu korrigierenden Bildwert der ersten oder zweiten Bildwerte und dem dazu örtlich korrespondierendem Bildwert der jeweils dem anderen Röntgenenergieschwellwert zugeordneten Bildwerte,

- Ausgeben (S3) des korrigierten Röntgenbilddatensatzes umfassend den zumindest einen korrigierten Bildwert der ersten oder zweiten Bildwerte.

FIG 1

EP 4 113 431 A1

**Beschreibung**

Die Erfindung betrifft ein Verfahren und eine Vorrichtung zur Ermittlung eines korrigierten Röntgenbilddatensatzes. Weiterhin betrifft die Erfindung ein Röntgenbildgebungssystem umfassend eine solche Vorrichtung, sowie ein Computerprogrammprodukt und ein Computerlesbares Speichermedium.

Mit Hilfe moderner bildgebender Verfahren werden häufig zwei- oder dreidimensionale Bilddaten erzeugt, die zur Visualisierung eines abgebildeten Untersuchungsobjekts und darüber hinaus auch für weitere Anwendungen genutzt werden können. Häufig basieren die bildgebenden Verfahren auf der Erfassung von Röntgenstrahlung, wobei sogenannte Projektionsdatensätze erzeugt werden. Beispielsweise können Projektionsdatensätze mit Hilfe eines Computertomographie-Geräts (CT-Gerät) akquiriert werden. Bei CT-Geräten läuft gewöhnlich eine an einer Gantry angeordnete Kombination aus Röntgenquelle und gegenüberliegend angeordnetem Röntgendetektor um einen Messraum um, in dem sich das Untersuchungsobjekt (das im Folgenden ohne Beschränkung der Allgemeinheit als Patient bezeichnet wird) befindet. Das Drehzentrum (auch "Isozentrum" genannt) fällt dabei mit einer sogenannten Systemachse, auch z-Achse genannt, welche sich in z-Richtung erstreckt, zusammen. Bei einem oder mehreren Umläufen wird der Patient mit Röntgenstrahlung der Röntgenquelle durchstrahlt, wobei mit Hilfe des gegenüberliegenden Röntgendetektors Projektionsdatensätze erfasst werden. Zur Rekonstruktion von zweidimensionalen oder dreidimensionalen, zur Visualisierung des abgebildeten Untersuchungsobjekts vorgesehenen Bilddatensätzen aus solchen Projektionsmessdaten, werden in der Computertomographie üblicherweise Rekonstruktionsalgorithmen der gefilterten Rückprojektion (FBP - Filtered Back Projection) eingesetzt. Daneben gibt es beispielsweise außerdem auch iterative Rekonstruktionsverfahren.

In der Röntgenbildgebung, d.h. auch in der Computertomographie (CT), aber ebenso auch in der der Angiographie oder der Radiographie, können photonenzählende, direkt-konvertierende Röntgendetektoren verwendet werden. Die Röntgenstrahlung oder die Photonen können in direkt-konvertierenden Röntgendetektoren durch ein geeignetes Konvertermaterial in elektrische Pulse umgewandelt werden. Als Konvertermaterial können beispielsweise CdTe, CZT, CdZnTeSe, CdTeSe, CdMnTe, InP, TlBr2, HgI2, GaAs oder andere verwendet werden. Die elektrischen Pulse können von elektronischen Schaltkreisen einer Auswerteeinheit, beispielsweise in Form eines integrierten Schaltkreises (Application Specific Integrated Circuit, ASIC), bewertet werden. In zählenden Röntgendetektoren kann die einfallende Röntgenstrahlung, d.h. die Intensität, insbesondere durch Zählen der elektrischen Pulse, welche durch die Absorption von Röntgenphotonen im Konvertermaterial ausgelöst werden, gemessen werden. Die Höhe des elektrischen Pulses ist in der Regel außerdem proportional zur Energie des absorbierten Röntgenphotons. Dadurch kann außerdem eine spektrale Information durch den Vergleich der Höhe des elektrischen Pulses mit einem Energieschwellwert extrahiert werden.

Werden zur Ermittlung der Projektionsdatensätze photonenzählende Röntgendetektoren eingesetzt, kann es dazu kommen, dass die ermittelten Bildwerte der Projektionsdatensätze von einem Photonenfluss abhängig sind. Je höher der am Detektor eintreffende Fluss, desto häufiger kommt es zu Überlagerungen von Ereignissen, sogenannten "pile-up" der im Röntgendetektor durch die Absorption von Photonen erzeugten Signalpulse. Dieser führt zu einer Verfälschung der gemessenen Anzahl an Photonen und ebenso auch der gemessenen Energie eines Photons und damit zu einem verfälschten Messergebnis, auf welchem die Bildwerte beruhen.

Eine Möglichkeit diesem Effekt entgegenzuwirken ist es, sicherzustellen, dass der Photonenfluss am Detektor niedrig genug und möglichst konstant ist. Praktisch ist dies in einem klinischen Betrieb aber nicht zuverlässig gewährleistet.

Der Erfindung liegt die Aufgabe zugrunde, ein Verfahren und eine Vorrichtung für die Ermittlung eines korrigierten Röntgenbilddatensatzes bereitzustellen, welcher verbessert, über einen größeren Bereich an Röntgenphotonenflüssen verlässliche und hochqualitative Röntgenbilddatensätze gewährleistet.

Die Aufgabe wird gelöst durch die Merkmale der unabhängigen Patentansprüche. Weitere vorteilhafte und teils für sich erfinderische Ausführungsformen und Weiterbildungen der Erfindung sind in den Unteransprüchen und der nachfolgenden Beschreibung dargelegt.

Die Erfindung betrifft ein Verfahren zur Ermittlung eines korrigierten Röntgenbilddatensatzes umfassend die Schritte

- Bereitstellen, mittels einer Schnittstelle, eines vorläufigen spektralen Röntgenbilddatensatzes darstellend eine ortsaufgelöste Abschwächung von Röntgenstrahlung beim Durchgang durch ein Objekt umfassend zumindest erste Bildwerte in Abhängigkeit eines ersten Röntgenenergieschwellwerts und zweite Bildwerte in Abhängigkeit eines zweiten Röntgenenergieschwellwerts,
- Korrigieren, mittels einer Recheneinheit, zumindest eines der ersten Bildwerte oder eines der zweiten Bildwerte des vorläufigen Röntgenbilddatensatzes mittels einer Skalierungsfunktion in Abhängigkeit eines Röntgenphotonenflusses und einer Kombination aus dem zu korrigierenden Bildwert der ersten oder zweiten Bildwerte und dem dazu örtlich korrespondierendem Bildwert der jeweils dem anderen Röntgenenergieschwellwert zugeordneten Bildwerte,
- Ausgeben, mittels einer zweiten Schnittstelle, des korrigierten Röntgenbilddatensatzes umfassend den zumindest einen korrigierten Bildwert der ersten oder zweiten Bildwerte.

Insbesondere kann der bereitgestellte spektrale Röntgenbilddatensatz mittels eines photonenzählenden Röntgendetektors ermittelt worden sein. Die ersten Bildwerte des vorläufigen Röntgenbilddatensatzes basieren dann insbesondere auf einer in Abhängigkeit des ersten Energieschwellwerts gezählten Anzahl an Röntgenphotonen, d.h. Signalpulsen, pro Ausleseintervall und Detektorpixelelement des Röntgendetektors. Die zweiten Bildwerte des vorläufigen Röntgenbilddatensatzes basieren dann insbesondere auf einer in Abhängigkeit des zweiten Energieschwellwerts gezählten Anzahl an Röntgenphotonen pro Ausleseintervall und Detektorpixelelement des Röntgendetektors. Die Bildwerte können logarithmierten Daten, wie sie in der Röntgenbildgebung üblich sind, entsprechen. Ein zu korrigierender Bildwert $S_{in}^t$, wobei $t$ die Zuordnung zu dem entsprechenden Röntgenenergieschwellwert beschreibt, kann folglich proportional zu einem Logarithmus aus der gezählten Anzahl S mit Objekt und der gezählten Anzahl $S_0$ ohne Objekt sein:

$$S_{in}^t \propto ln(S/S_0)$$

Die Bildwerte können dann insbesondere als Schätzungen für die Linienintegrale der Absorptionskoeffizienten durch das Objekt verstanden werden.

Der vorläufige Röntgenbilddatensatz ist ein spektraler Röntgenbilddatensatz in dem Sinne, dass Bildwerte in Abhängigkeit von den zumindest zwei Röntgenenergieschwellwerten bereitgestellt werden. Der vorläufige Röntgenbilddatensatz kann dann im Wesentlichen eine erste Röntgenschwächungsverteilung anhand der ersten Bildwerte, d.h. darstellend eine ortsaufgelöste Abschwächung von Röntgenstrahlung beim Durchgang durch das Objekt, in Abhängigkeit einer ersten Röntgenquantenenergieverteilung und eine zweite Röntgenschwächungsverteilung anhand der zweiten Bildwerte, d.h. darstellend eine ortsaufgelöste Abschwächung von Röntgenstrahlung beim Durchgang durch das Objekt, in Abhängigkeit einer zweiten Röntgenquantenenergieverteilung umfassen, wobei die erste und die zweite Röntgenquantenenergieverteilung durch die Röntgenenergieschwellwerte bestimmt werden.

Es ist bekannt, dass unterschiedliche Materialien bzw. Gewebetypen, beispielsweise Wasser, Knochen oder ein appliziertes Kontrastmittel, unterschiedlich stark mit Röntgenstrahlung wechselwirken. Darüber hinaus ist auch die Energie-Abhängigkeit der Röntgenschwächung beim Durchlaufen von Materie bekannt. Das bedeutet, dass niederenergetische Röntgenstrahlung stärker von Materie absorbiert wird als höherenergetische Röntgenstrahlung. Durch ein in Beziehung setzen der ersten und der dazu örtlich korrespondierenden zweiten Bildwerte des vorläufigen Röntgenbilddatensatzes kann daher Rückschlüsse auf im Objekt vorliegende Materialien zulassen und eine Korrektur, welche diese berücksichtigt. Insbesondere bei stark röntgensensitiven Materialien, wie beispielsweise ein Kontrastmittel, aber auch Knochen, kann sich ein erster Bildwert und ein dazu örtlich korrespondierender zweiter Bildwert des vorläufigen Röntgenbilddatensatzes deutlich stärker unterscheiden als beispielsweise für fetthaltige und wasserhaltige Gewebe.

Der vorläufige und entsprechend der korrigierte Röntgenbilddatensatz kann insbesondere ein Computertomographie-Bilddatensatz sein. Insbesondere kann der vorläufige und entsprechend der korrigierte Röntgenbilddatensatz ein Projektionsdatensatz sein, d.h. ein Messdatensatz, welcher aus einer Winkelrichtung relativ zu dem Objekt die ortsaufgelöste Abschwächung von Röntgenstrahlung beim Durchgang durch das Objekt abbildet. Die Anwendung des Verfahrens in der Computertomographie ist dabei besonders vorteilhaft, da hier besonders hohe Photonenflüsse angewendet werden, so dass die aufgenommenen Datensätze besonders durch Effekte wie "pile-up" beeinträchtigt sein können. Ein solcher Projektionsdatensatz kann auch als Rohbilddatensatz bezeichnet werden. Dies kann jedoch umfassen, dass auch vor der Korrektur bereits andere Verarbeitungsschritte, beispielsweise anderweitige Korrekturen wie etwa eine Streustrahlenkorrektur oder eine Aufhärtungskorrektur angewendet wurden.

Das Verfahren kann jedoch auch in anderen Röntgenanwendungen, beispielsweise im Rahmen einer Bildgebung mittels eines C-Bogen-Röntgengerät oder in der Mammographie, eingesetzt werden.

Im Rahmen des erfindungsgemäßen Verfahrens wird zumindest einer der ersten Bildwerte oder einer der zweiten Bildwerte des vorläufigen Röntgenbilddatensatzes mittels einer Skalierungsfunktion in Abhängigkeit eines Röntgenphotonenflusses und einer Kombination aus dem zu korrigierenden Bildwert der ersten oder zweiten Bildwerte und dem dazu örtlich korrespondierendem Bildwert der jeweils dem anderen Röntgenenergieschwellwert zugeordneten Bildwerte korrigiert. Dies kann umfassen, dass sowohl ein Bildwert der ersten Bildwerte als auch ein Bildwert der zweiten Bildwerte korrigiert wird. Dabei unterscheidet sich in der Regel zumindest ein Parameter der Skalierungsfunktion für den zu korrigierenden Bildwert der ersten Bildwerte und für den zu korrigierenden Bildwert der zweiten Bildwerte. Insbesondere kann auch auf jeden der ersten und/oder jeden der zweiten Bildwerte eine Skalierungsfunktion angewendet werden um korrigierte erste und/oder zweite Bildwerte zu ermitteln, wobei die Stärke der Korrektur durch die Skalierungsfunktion bestimmt ist.

Die Abhängigkeit von einem Photonenfluss kann insbesondere dergestalt sein, dass bei einem höheren Photonenfluss eine stärkere Korrektur erfolgt als bei einem relativ dazu niedrigeren Photonenfluss entsprechend der vermehrt auftretenden Überlagerungen von Signalpulsen bei hohen Flüssen, so dass eine Abhängigkeit vermieden oder zumindest reduziert werden kann. Der oben beschriebene Effekt der Pulsüberlagerung ist abhängig vom am Detektor eintreffenden Photonenfluss, welcher insbesondere abhängig von dem durch die zur Aufnahme des Röntgenbilddatensatzes verwendeten Röntgenquelle emittierten Photonenfluss. Weiterhin wird der am Röntgendetektor eintreffende Photonenfluss von der örtlichen Abschwächung durch das Objekt, welcher sich in den Bildwerten widerspiegelt, in den Strahlengang eingeführter Filter, beispielsweise ein Formfilter oder ein spektraler Filter beeinflusst. Der von der verwendeten Röntgenröhre emittierten Photonenfluss ist dabei direkt abhängig von dem zur Aufnahme der Daten verwendeten Röntgenröhrenstrom.

Die Kombination aus dem zu korrigierenden Bildwert der ersten oder zweiten Bildwerte und dem dazu örtlich korrespondierendem Bildwert der jeweils dem anderen Röntgenenergieschwellwert zugeordneten Bildwerte kann umfassen, dass der eine zu korrigierende Bildwert mit dem dazu örtlich korrespondierendem Bildwert der jeweils dem anderen Röntgenenergieschwellwert zugeordneten Bildwerte verrechnet wird. Durch die Kombination wird ein Zusammenhang zwischen dem zu korrigierenden Bildwert der ersten oder zweiten Bildwerte und dem dazu örtlich korrespondierendem Bildwert der jeweils dem anderen Röntgenenergieschwellwert zugeordneten Bildwerte hergestellt. Insbesondere kann die Kombination ein Unterschied zwischen dem zu korrigierende Bildwert mit dem dazu örtlich korrespondierendem Bildwert der jeweils dem anderen Röntgenenergieschwellwert zugeordneten Bildwerte widerspiegeln.

Indem die Skalierungsfunktion eine Abhängigkeit von der Kombination, vorzugsweise einer Differenz oder eines Quotienten, aus dem zu korrigierenden Bildwert der ersten oder zweiten Bildwerte und dem dazu örtlich korrespondierendem Bildwert der jeweils dem anderen Röntgenenergieschwellwert zugeordneten Bildwerte aufweist, kann vorteilhafterweise eine Objektinformation, welche über die Bildwerte an sich hinausgeht, in die Skalierungsfunktion aufgenommen werden. Diese kann Information über das im Objekt vorliegende Material bereitstellen. Derart ist eine Skalierung, d.h. Korrektur, in Abhängigkeit dieser Objektinformation ermöglicht.

Derart kann erreicht werden, dass insbesondere für ein bestimmtes Material oder zumindest bevorzugt für ein bestimmtes Material eine Korrektur erfolgt, so dass ein Bildwert, darstellend die Abschwächung durch dieses Material im Körper, unabhängig von dem Photonenfluss ist, wohingegen andere Materialien weniger stark von der Korrektur beeinflusst sind. Dies erlaubt eine optimierte Anpassung der Korrektur auf das interessierende Material ohne die Messwerte in anderen Bereichen, auf welchen die Skalierung ggf. nicht optimiert ist, unnötig zu beeinflussen und ggf. zu verfälschen.

Beispielsweise ist der Röntgenbilddatensatz ein kontrastmittelverstärkter Röntgenbilddatensatz. Ein kontrastmittelverstärkter Röntgenbilddatensatz kann durch Injektion eines Kontrastmittels, beispielweise Jod , während der Erfassung der Daten mittels eines Röntgendetektors erzeugt werden. Insbesondere bei der Evaluation von kontrastmittelverstärkten Bilddatensätzen kann sich für eine quantitative Bestimmung und Beurteilung der Kontrastmittelkonzentration im Objekt eine Flussabhängigkeit der Bildwerte besonders negativ auswirken. Vorteilhaft kann eine Korrektur mittels einer erfindungsgemäßen Skalierungsfunktion, welche sowohl den Photonenfluss als auch einen ersten und einen zweiten Bildwert, insbesondere einen Unterschied zwischen dem ersten und dem zweiten Bildwert berücksichtigt, ermöglichen, insbesondere diejenigen ersten und/oder zweiten Bildwerte optimal zu korrigieren, welche durch das Kontrastmittel im Objekt beeinflusst sind. Damit kann eine quantitative Untersuchung verbessert ermöglicht werden.

Beispielsweise sind die Parameter der Skalierungsfunktion auf die Anwendung auf kontrastmittelverstärkte Bilddatensätze und das Ziel der Bereitstellung eines möglichst vom Photonenfluss unabhängigen Bildwerts für das Kontrastmittel optimiert. Dies kann beispielsweise dadurch gewährleistet werden, dass für die experimentelle Bestimmung von Parametern der Skalierungsfunktion kontrastmittelverstärkte Röntgenbilddatensätze herangezogen werden.

Gemäß einer vorteilhaften Verfahrensvariante entspricht die Kombination aus sowohl dem zu korrigierenden Bildwert als auch dem dazu örtlich korrespondierendem Bildwert der jeweils dem anderen Röntgenenergieschwellwert zugeordneten Bildwerte einer Differenz der jeweiligen Bildwerte oder einem Quotienten aus den jeweiligen Bildwerten. Eine Differenz oder ein Quotient kann charakteristisch für ein Material und dessen Konzentration im Objekt sein. Insbesondere zeigen röntgensensitive Materialien, beispielsweise Kontrastmittel, einen besonders großen Unterschied zwischen einem jeweiligen Bildwert der ersten Bildwerte und dem zugehörigen, örtlich korrespondierenden Bildwert der zweiten Bildwerte, welcher sich vorteilhaft in einer Differenz oder einem Quotienten widerspiegelt. Vorteilhaft kann die Korrekturstärke anhand einer Differenz oder eines Quotienten einfach und effizient auf ein Material, insbesondere ein Kontrastmittel, abgestimmt werden.

Weiterhin kann es vorteilhaft sein, wenn die Korrektur zumindest des einen der ersten Bildwerte oder des einen der zweiten Bildwerte nach einer Wasserskalierung der ersten oder zweiten Bildwerte durchgeführt wird. Dies gilt insbesondere im Rahmen von Computertomographie-Bilddatensätzen, bei welchen eine Wasserskalierung wie üblich bekannt durchgeführt wird. Die Anwendung der Korrektur nach der Wasserskalierung kann vorteilhaft gewährleisten, dass die Korrektur für Bildwerte darstellend Wasser oder Weichteilgewebe nur eine geringe Anpassung erzeugt, da die Wasserskalierung im Wesentlichen bewirkt, dass diese in allen Röntgenbilddatensätzen, unabhängig von dem Röntgenenergieschwellwert oder anderen Parametern einen ähnlichen oder gleichen Bildwert aufweisen.

In einer bevorzugten Verfahrensvariante ist die Skalierungsfunktion abhängig von zumindest einem der folgenden Größen:

- einem Einstellungsparameter einer zur Aufnahme des spektralen Röntgenbilddatensatzes eingesetzten Röntgenquelle,
- einem Formfilterparameter eines zur Aufnahme des spektralen Röntgenbilddatensatzes eingesetzten Formfilters,
- einem Filterparameter eines zur Aufnahme des spektralen Röntgenbilddatensatzes eingesetzten spektralen Filters, oder
- dem ersten oder zweiten Röntgenenergieschwellwert.

Ein Einstellungsparameter einer zur Aufnahme des spektralen Röntgenbilddatensatzes eingesetzten Röntgenröhre, kann neben dem Röntgenröhrenstrom beispielsweise außerdem eine verwendete Röntgenröhrenspannung sein. Die Röntgenröhrenspannung hat Einfluss auf die emittierte Verteilung an Röntgenquantenenergien und damit auch auf die resultierende Abschwächung durch das Objekt. Weiterhin tritt der Effekt der Überlagerung von Signalpulsen bei höheren Röntgenquantenenergien relativ zu niedrigeren Röntgenquantenenergien verstärkt auf.

Ein Formfiltern (engl. häufig "bow-tie filter") kann zur Dosisreduktion während des Erfassens der Daten eingesetzt werden. Dieser kann insbesondere ortsabhängig eine zusätzliche Röntgenabschwächung bewirken, beispielsweise kann ein solcher Filter die Strahlung zentral nur minimal abschwächen, in der Peripherie hingegen stark. Der Formfilter hat damit Einfluss auf den Photonenfluss. Außerdem kann der Formfilter auch einen Einfluss auf die emittierte Röntgenenergieverteilung durch ortsabhängige Aufhärtung der Strahlung bewirken.

Ein spektraler Filter kann eingesetzt werden, um die von der Röntgenquelle emittierte Röntgenenergieverteilung vor Durchgang durch den Patienten anzupassen. Häufig werden dazu Filter aus Kupfer oder Zinn verwendet. Ein solcher spektraler Filter kann außerdem auch Einfluss auf den Photonenfluss haben.

In einer vorteilhaften Ausführungsvariante umfasst die Skalierungsfunktion eine Exponentialfunktion oder ein Polynom höherer Ordnung. Die Skalierungsfunktion kann auch eine Exponentialfunktion und ein Polynom höherer Ordnung umfassen.

Weiterhin kann es vorteilhaft sein, wenn die Korrektur erst ab einem minimalen Röntgenphotonenfluss durchgeführt wird. Eine Korrektur bei niedrigen Flüssen kann unnötig sein, da hier ein geringerer Effekt durch pile-up zu erwarten ist. Dies kann insbesondere vorteilhaft zur Zeiteffizienz des Verfahrens beitragen.

In einer Verfahrensvariante, in welcher eine Mehrzahl an spektralen Röntgenbilddatenätzen unter Verwendung unterschiedlicher Röntgenphotonenflüsse bereitgestellt wird, kann daher vorteilhaft für jeden der bereitgestellten spektralen Röntgenbilddatensätze jeweils der Schritt des Korrigierens durchgeführt werden. Dadurch kann sichergestellt werden, dass auch bei variierenden Photonenflüssen verlässliche und von Röntgenbilddatensatz zu Röntgenbilddatensatz vergleichbarere Bildwerte ermittelt werden können.

Insbesondere in der Computertomographie kann es häufig vorkommen, dass für aufeinanderfolgende Röntgenbilddatensätze unterschiedliche Photonenflüsse verwendet werden, so dass hier der Effekt einer Abhängigkeit vom Photonenfluss besonders nachteilig auf die erzielten Ergebnisse wirken kann. Ein solches Vorgehen ist in der Computertomographie unter dem Begriff Dosismodulation bekannt und dient der Gewährleistung einer möglichst niedrigen applizierten Dosis.

Die Erfindung betrifft weiterhin eine Vorrichtung zur Ermittlung eines korrigierten Röntgenbilddatensatzes eines Objekts umfassend

- eine erste Schnittstelle ausgebildet zum Bereitstellen eines vorläufigen spektralen Röntgenbilddatensatzes umfassend zumindest erste Bildwerte in Abhängigkeit eines ersten Röntgenenergieschwellwerts und zweite Bildwerte in Abhängigkeit eines zweiten Röntgenenergieschwellwerts,
- eine Recheneinheit ausgebildet zum Korrigieren zumindest eines der ersten Bildwerte oder eines der zweiten Bildwerte des vorläufigen Röntgenbilddatensatzes mittels einer Skalierungsfunktion in Abhängigkeit eines Röntgenphotonenflusses und einer Kombination aus sowohl dem zu korrigierenden Bildwert als auch dem dazu örtlich korrespondierendem Bildwert der jeweils dem anderen Röntgenenergieschwellwert zugeordneten Bildwerte, und
- eine zweite Schnittstelle ausgebildet zum Ausgeben des korrigierten Röntgenbilddatensatzes umfassend den zumindest einen korrigierten Bildwert der ersten oder zweiten Bildwerte.

Eine solche Vorrichtung zur Ermittlung eines korrigierten Röntgenbilddatensatzes kann insbesondere dazu ausgebildet sein, die zuvor beschriebenen erfindungsgemäßen Verfahren zur Ermittlung eines korrigierten Röntgenbilddatensatzes und ihre Aspekte auszuführen. Die Vorrichtung kann dazu ausgebildet sein, die Verfahren und ihre Aspekte auszuführen, indem die Schnittstellen und die Recheneinheit ausgebildet sind, die entsprechenden Verfahrensschritte auszuführen.

Die Vorteile der vorgeschlagenen Vorrichtung entsprechen im Wesentlichen den Vorteilen des vorgeschlagenen Verfahrens zur Ermittlung eines korrigierten Röntgenbilddatensatzes. Hierbei erwähnte Merkmale, Vorteile oder alternative Ausführungsformen können ebenso auch auf die Vorrichtung zur Ermittlung eines korrigierten Röntgenbilddatensatzes übertragen werden und umgekehrt.

Die Erfindung betrifft außerdem ein Röntgenbildgebungssystem umfassend eine Vorrichtung zur Ermittlung eines korrigierten Röntgenbilddatensatzes wie zuvor beschrieben und eine Röntgenquelle in Gegenüberstellung zu einem Röntgendetektor, wobei zwischen die Röntgenquelle und den Röntgendetektor zur Aufnahme des spektralen Röntgenbilddatensatz ein Objekt platzierbar ist.

Dabei ist das Röntgenbildgebungssystem vorteilhafterweise zur Ausführung einer Ausführungsform des vorgeschlagenen Verfahrens zur Ermittlung eines korrigierten Röntgenbilddatensatzes ausgebildet. Die Vorteile des vorgeschlagenen Röntgenbildgebungssystem entsprechen im Wesentlichen den Vorteilen des vorgeschlagenen Verfahrens zur Ermittlung eines korrigierten Röntgenbilddatensatzes. Hierbei erwähnte Merkmale, Vorteile oder alternative Ausführungsformen können ebenso auch auf das Röntgenbildgebungssystem übertragen werden und umgekehrt.

Das Röntgenbildgebungssystem kann vorzugsweise als Computertomographie-Gerät ausgebildet sein.

In anderen Ausbildungen kann das Röntgenbildgebungssystem auch ein anderweitiges Bildgebungssystem basierend auf Röntgenstrahlung sein. Beispielsweise kann das Röntgenbildgebungssystem ein C-Bogen-Röntgengerät oder ein Mammographie-Gerät oder ein Röntgenbildgebungssystem für die Radiographie sein.

Weiterhin betrifft die Erfindung ein Computerprogrammprodukt mit einem Computerprogramm, welches direkt in einen Speicher einer Vorrichtung zur Ermittlung eines korrigierten Röntgenbilddatensatzes ladbar ist, mit Programmabschnitten, um alle Schritte eines der zuvor beschriebenen Verfahrenen zur Ermittlung eines korrigierten Röntgenbilddatensatzes oder ihre Aspekte auszuführen, wenn die Programmabschnitte von der Vorrichtung ausgeführt werden.

Ein Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist so konfiguriert, dass es mittels der Vorrichtung die erfindungsgemäßen Verfahrensschritte ausführen kann. Die Vorrichtung muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in eine Recheneinheit der Vorrichtung geladen werden kann.

Die Erfindung kann weiterhin ein computerlesbares Speichermedium betreffen, auf welchem von der Vorrichtung lesbare und ausführbare Programmabschnitte gespeichert sind, um alle Schritte eines der zuvor beschriebenen Verfahrenen zur Ermittlung eines korrigierten Röntgenbilddatensatzes oder ihre Aspekte auszuführen, wenn die Programmabschnitte von der Vorrichtung ausgeführt werden.

Beispiele für ein computerlesbares Speichermedium sind eine DVD, ein Magnetband, eine Festplatte oder ein USB-Stick, auf welchem elektronisch lesbare Steuerinformationen, insbesondere Software, gespeichert ist.

Eine weitgehend softwaremäßige Realisierung hat den Vorteil, dass auch schon bisher verwendete Verarbeitungseinheiten auf einfache Weise durch ein Software-Update nachgerüstet werden können, um auf die erfindungsgemäße Weise zu arbeiten. Ein Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen

Im Rahmen der Erfindung können außerdem Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist das Röntgenbildgebungssystem das Röntgenbildgebungssystem auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden wird die Erfindung anhand von beispielhaften Ausführungsformen unter Hinweis auf die beigefügten Figuren erläutert. Die Darstellung in den Figuren ist schematisch, stark vereinfacht und nicht zwingend maßstabsgetreu. Es zeigen:

Fig. 1 einen schematischen Verfahrensablauf eines Verfahrens zur Ermittlung eines korrigierten Röntgenbilddatensatzes,

Fig. 2 eine schematische Darstellung einer Vorrichtung zur Ermittlung eines korrigierten Röntgenbilddatensatzes, und

Fig. 3 eine schematische Darstellung eines Röntgenbildgebungssystems.

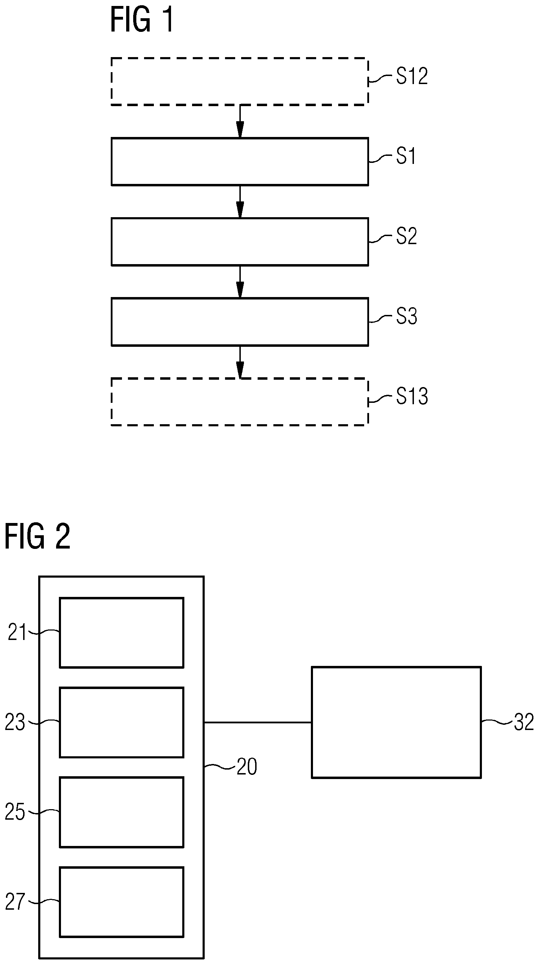

Fig. 1 zeigt ein Verfahren zur Ermittlung eines korrigierten Röntgenbilddatensatzes umfassend die Schritte

- Bereitstellen S1, mittels einer ersten Schnittstelle 21, eines vorläufigen spektralen Röntgenbilddatensatzes darstellend eine ortsaufgelöste Abschwächung von Röntgenstrahlung beim Durchgang durch ein Objekt 39 umfassend zumindest erste Bildwerte in Abhängigkeit eines ersten Röntgenenergieschwellwerts und zweite Bildwerte in Abhängigkeit eines zweiten Röntgenenergieschwellwerts,
- Korrigieren S2, mittels einer Recheneinheit 23, zumindest eines der ersten Bildwerte oder eines der zweiten Bildwerte des vorläufigen Röntgenbilddatensatzes mittels einer Skalierungsfunktion in Abhängigkeit eines Röntgenphotonenflusses und einer Kombination aus dem zu korrigierenden Bildwert der ersten oder zweiten Bildwerte und dem dazu örtlich korrespondierendem Bildwert der jeweils dem anderen Röntgenenergieschwellwert zugeordneten Bildwerte,
- Ausgeben S3, mittels einer zweiten Schnittstelle 25, des korrigierten Röntgenbilddatensatzes umfassend den zumindest einen korrigierten Bildwert der ersten oder zweiten Bildwerte.

Vor dem Bereitstellen S1 kann bereits eine Vorverarbeitung des Röntgenbilddatensatzes bzw. der Bildwerte durchgeführt werden. Beispielsweise kann ein Schritt S12 weitere Korrekturen, etwa eine Streustrahlenkorrektur, eine Strahlaufhärtungskorrektur, wie sie üblicherweise bekannt sind, oder ähnliches umfassen. Insbesondere kann eine Wasserskalierung sowohl der ersten Bildwerte als auch der zweiten Bildwerte durchgeführt werden.

Nach der Ausgabe des korrigierten Röntgenbilddatensatzes können sich ebenfalls weitere Verarbeitungsschritte anschließen. Insbesondere kann im Falle eines Computertomographie-Röntgenbilddatensatzes eine Rekonstruktion, beispielsweise mittels eines Verfahren der gefilterten Rückprojektion, durchgeführt werden.

Im Folgenden werden konkrete, beispielhafte Implementierungen der Skalierungsfunktion beschrieben, welche zu einem vorteilhaften Ergebnis führen können. Daneben kann es auch weitere erfindungsgemäße Implementierungen geben.

Im Folgenden wird von einem Computertomographie-Bilddatensatz, insbesondere einen Projektionsdatensatz umfassend die ersten und die zweiten Bildwerte, ausgegangen, wobei die Korrektur auf logarithmierten Daten, wie sie in der Computertomographie-Bildgebung üblich sind, angewendet wird. Weiterhin wird angenommen, dass zumindest eine Wasserskalierung der ersten und der zweiten Bildwerte für besonders vorteilhafte Ergebnisse des erfindungsgemäßen Verfahrens durchgeführt wurde.

Gemäß einer Variante kann eine mögliche Implementierung der Skalierungsfunktion für den einen der ersten Bildwerte und den einen der zweiten Bildwerte in der folgenden Art realisiert sein:

$$S^1_{\text{out}} = S^1_{in} + \epsilon_1 \cdot (S^1_{in} - S^2_{in})$$

$$S^2_{\text{out}} = S^2_{in} + \epsilon_2 \cdot (S^1_{in} - S^2_{in})$$

dabei entspricht $S^1_{in}$ dem zu korrigierenden Bildwert der ersten Bildwerte, welcher dem ersten Röntgenenergieschwellwert $t = 1$ zugeordnet ist, und $S^2_{in}$ dem dazu örtlich korrespondierenden Bildwert der zweiten Bildwerte, welcher dem zweiten Röntgenenergieschwellwert $t = 2$ zugeordnet ist.

Die Korrektur ist abhängig von einer Kombination, hier insbesondere der Differenz, aus dem zu korrigierenden Bildwert $S^1_{in}, S^2_{in}$ der ersten bzw. zweiten Bildwerte und dem dazu örtlich korrespondierendem Bildwert $S^2_{in}, S^1_{in}$ der jeweils dem anderen Röntgenenergieschwellwert zugeordneten Bildwerte. Je größer die Differenz, desto stärker ist der Beitrag des zweiten Terms und damit die Korrekturstärke. Ist der zu korrigierende erste Bildwert gleich dem örtlich korrespondierenden zweiten Bildwert erfolgt keine Korrektur. Statt einer Differenz könnte auch eine anderweitige Kombination gewählt sein, welche insbesondere den einen der ersten Bildwerte und den einen der zweiten Bildwerte ins Verhältnis setzt.

$\varepsilon_1$ bzw. $\varepsilon_2$ sind Skalierungsfaktoren, welche dem ersten bzw. zweiten Röntgenenergieschwellwert zugeordnet

sind. Die Skalierungsfaktoren $\varepsilon_t$ können insbesondere unterschiedlich für den einen der ersten Bildwerte und den einen der zweiten Bildwerte sein.

In einem ersten Beispiel können die Skalierungsfaktoren etwa im Wesentlichen eine folgende Abhängigkeit aufweisen:

$$\epsilon_t = -abs(\alpha_t \cdot exp(-S_{in}^1) \cdot (M + \beta_t)^x),$$

wobei $x \geq 1$, beispielsweise 3 oder 4. Dabei sind $\alpha_t$ und $\beta_t$ experimentell bestimmte Parameter in Abhängigkeit der ersten bzw. zweiten Röntgenenergieschwellwerte $t$ = 1,2. M ist abhängig von einem von der zur Aufnahme verwendeten Röntgenquelle emittierten Röntgenphotonenfluss. Dieser kann beispielsweise direkt mit einem eingestellten Röhrenstrom zusammenhängen, welcher für die Aufnahme der ersten bzw. zweiten Bildwerte verwendet wurde. M kann außerdem bereits den Einfluss von etwaigen eingebrachten Filtern umfassen, insofern der Photonenfluss nach Durchgang durch solche Filter ermittelt wurde. $\alpha_t$ und $\beta_t$ können ebenso beispielsweise beeinflusst sein von weiteren Aufnahmeparametern, beispielsweise einer Röntgenröhrenspannung, einem Parameter eines eingesetzten Formfilters oder eines spektralen Filters, beispielsweise einem Zinn- oder Kupferfilter, welcher für die Ermittlung der Daten in den Strahlengang der verwendeten Röntgenquelle eingeführt wurde. $\alpha_t$ und $\beta_t$ können insbesondere außerdem auf eine bestimmte Anwendung optimiert sein, beispielsweise für ein bestimmtes Kontrastmittel. $\alpha_t$ und $\beta_t$ können durch vorab durchgeführte Kalibrationsmessungen bestimmt sein und im Form einer Lookup-Tabelle für verschiedene Aufnahmeparameter für ein Abrufen, ggf. in Abhängigkeit einer gewählten Anwendung, bereitgestellt werden. Der Parameter M könnte beispielsweise auf einem während der Aufnahme durchgeführten Dosismonitoring basieren oder anhand des eingestellten Röhrenstroms und der bekannten anderen Aufnahmeparametern, wie eingeführter Filter, berechnet oder ebenfalls anhand einer Lookup-Tabelle bestimmt werden.

Im Falle, dass $\varepsilon_t$ = 0 erfolgt keine Korrektur. Weiterhin nimmt die Stärke der Korrektur zu, je größer der Photonenfluss, repräsentiert durch den Faktor M, und je geringer eine ermittelte Abschwächung ist.

In einem zweiten Beispiel können die Skalierungsfaktoren auch aus einer folgenden Abhängigkeit bestimmt sein

$$\epsilon_t = -\gamma_t \cdot (I - I_{min,t}) \cdot exp\left(-(S_{in}^1 + W_t)\right)$$

mit $\gamma_t$ einem experimentell bestimmten Parameter in Abhängigkeit der ersten bzw. zweiten Röntgenenergieschwellwerte $t$ = 1 bzw.2, $W_t$ einem optionalen Faktor, welcher eine resultierende Abschwächung durch einen in den Strahlengang optional eingebrachten Formfilter widerspiegelt. I entspricht dem für die Erfassung der Bildwerte verwendeten Röntgenröhrenstrom und ist damit direkt mit dem von der Röntgenröhre emittierten Röntgenphotonenfluss gekoppelt. $I_{min,t}$ entspricht einem vom Röntgenenergieschwellwert abhängigen unteren Limit. $\gamma_t$ kann insbesondere abhängig sein von weiteren Aufnahmeparametern, beispielsweise einer verwendeten Röntgenröhrenspannung, einem eingesetzten Formfilter oder einem spektralen Filter. $\gamma_t$ kann genauso wie die oben beschriebenen Parameter $\alpha_t$ und $\beta_t$ bestimmt sein, beispielsweise durch vorab durchgeführte Kalibrationsmessungen. $W_t$ kann aus der bekannten Ausgestaltung des Formfilters und den verwendeten, bekannten Aufnahmeparametern bestimmt werden.

Die Abhängigkeit direkt vom Röntgenröhrenstrom ist insbesondere vorteilhaft, da dies einem leicht zugänglichen Parameter entspricht. Weiterhin ist der Formfilter und dessen Abschwächungsverhalten bekannt und kann relativ einfach integriert werden.

In beiden beschriebenen Beispielen können auch noch weitere Parameter oder Skalierungsfaktoren umfasst sein.

Es kann außerdem vorgesehen sein, dass, wenn der verwendete Röntgenröhrenstrom I unter dem vom Röntgenenergieschwellwert abhängigen unteren Limit $I_{min,t}$ liegt, der jeweilige Skalierungsfaktor $\varepsilon_t$ einen Grenzwert annimmt. Es kann bei der Berechnung der jeweiligen korrigierten Bildwerte beispielsweise folgende Bedingung angewendet werden:

$$wenn\ I < I_{min,t}: \epsilon_t = 0$$

In diesem Fall erfolgt unterhalb des gewählten Limits, welches einen minimalen von der Röntgenröhre emittierten Röntgenphotonenfluss widerspiegelt, keine Korrektur des zu korrigierenden Bildwerts. Da das Limit für den einen der ersten und den einen der zweiten Bildwerte unterschiedlich sein kann, kann hier insbesondere vorkommen, dass nur der eine Bildwert der ersten Bildwerte und nicht der eine Bildwert der zweiten Bildwerte korrigiert wird oder andersherum. In anderen Ausführungsvarianten kann eine andere Implementierung, beispielsweise ein von Null abweichender Grenzwert für den Betrag des Skalierungsfaktor $\varepsilon_t$, gewählt sein.

Geht in die Skalierungsfunktion nicht der Röntgenröhrenstrom selbst als Parameter ein, kann ohne weiteres eine ähnliche Bedingung auch für einen anderen den Röntgenphotonenfluss betreffenden Parameter definiert werden, so dass die Korrektur erst ab einem minimalen Röntgenphotonenfluss durchgeführt wird. Es kann auch kein unteres Limit hinsichtlich des Röntgenphotonenflusses vorgesehen sein.

Weiterhin kann vorgesehen sein, dass ein weiterer Grenzwert $C_t$ hinsichtlich eines maximalen Betrags für einen Skalierungsfaktor $\varepsilon_t$ vorgesehen ist:

$$\epsilon_t = max(\epsilon_t, C_t)$$

Der Grenzwert kann insbesondere abhängig vom Röntgenenergieschwellwert sein und entsprechend verschieden für den einen zu korrigierenden Bildwert der ersten Bildwerte und den einen zu korrigierenden Bildwert der zweiten Bildwerte. Ein solcher Grenzwert hinsichtlich eines maximalen Betrags kann vorteilhaft eine Überkorrektur vermeiden helfen.

Fig. 2 zeigt eine schematische Darstellung einer Vorrichtung 20 zur Ermittlung eines korrigierten Röntgenbilddatensatzes eines Objekts 39 umfassend

- eine erste Schnittstelle 21 ausgebildet zum Bereitstellen eines vorläufigen spektralen Röntgenbilddatensatzes umfassend zumindest erste Bildwerte in Abhängigkeit eines ersten Röntgenenergieschwellwerts und zweite Bildwerte in Abhängigkeit eines zweiten Röntgenenergieschwellwerts,
- eine Recheneinheit 23 ausgebildet zum Korrigieren zumindest eines der ersten Bildwerte oder eines der zweiten Bildwerte des vorläufigen Röntgenbilddatensatzes mittels einer Skalierungsfunktion in Abhängigkeit eines Röntgenphotonenflusses und einer Kombination aus sowohl dem zu korrigierenden Bildwert als auch dem dazu örtlich korrespondierendem Bildwert der jeweils dem anderen Röntgenenergieschwellwert zugeordneten Bildwerte, und
- eine zweite Schnittstelle 25 ausgebildet zum Ausgeben des korrigierten Röntgenbilddatensatzes umfassend den zumindest einen korrigierten Bildwert der ersten oder zweiten Bildwerte.

Die Vorrichtung 20 kann außerdem eine Speichereinheit 27 umfassen.

Die Vorrichtung 20 ist insbesondere mit dem Röntgenbildgebungssystem 32 signaltechnisch gekoppelt, so dass das Röntgenbildgebungssystem 32 mittels der Vorrichtung 20 ansteuerbar ist.

Die Vorrichtung 20 ist insbesondere zur Ausführung eines vorgeschlagenen Verfahrens zur Ermittlung eines korrigierten Röntgenbilddatensatzes und seiner Aspekte ausgebildet.

Bei der Vorrichtung 20 kann es sich insbesondere um einen Computer, einen Mikrocontroller oder um einen integrierten Schaltkreis handeln. Alternativ kann es sich dabei um einen realen oder virtuellen Verbund von Computern handeln (ein englischer Fachbegriff für einen realen Verbund ist "Cluster", ein englischer Fachbegriff für einen virtuellen Verbund ist "Cloud"). Die Vorrichtung 20 kann auch als virtuelles System ausgebildet sein, das auf einem realen Computer oder einem realen oder virtuellen Verbund von Computern ausgeführt wird (engl. virtualization).

Bei einer Schnittstelle 21, 25 kann es sich um eine Hardware- oder Softwareschnittstelle handeln (beispielsweise PCI-Bus, USB oder Firewire). Eine Recheneinheit 23 kann Hardware-Elemente oder Software-Elemente aufweisen, beispielsweise einen Mikroprozessor oder ein sogenanntes FPGA (englisches Akronym für "Field Programmable Gate Array"). Eine Speichereinheit 27 kann als nicht dauerhafte Arbeitsspeicher (Random Access Memory, kurz RAM) oder als dauerhafter Massenspeicher (Festplatte, USB-Stick, SD-Karte, Solid State Disk) realisiert sein.

Die Schnittstelle 21, 25 können insbesondere mehrere Unterschnittstellen umfassen. Mit anderen Worten kann die Schnittstelle 21,25 auch eine Vielzahl von Schnittstellen 21,25 umfassen. Die Recheneinheit 23 kann insbesondere mehrere Unterrecheneinheiten umfassen, die unterschiedliche Schritte der jeweiligen Verfahren ausführen. Mit anderen Worten kann die Recheneinheit 20 auch als Vielzahl von Recheneinheiten 20 aufgefasst werden.

Im gezeigten Ausführungsbeispiel ist die Vorrichtung S20 direkt mit einem Röntgenbildgebungssystem 32 gekoppelt. Insbesondere kann die Vorrichtung auch von dem Röntgenbildgebungssystem 32 umfasst sein. Die Vorrichtung 20 kann aber auch mittels des Netzwerks NETW mit dem Röntgenbildgebungssystem 32 verbunden sein. Weiterhin kann eine Kommunikation auch offline erfolgen, beispielsweise durch einen Austausch von Datenträgern.

Beim Netzwerk NETW kann es sich um ein lokales Netzwerk (ein englischer Fachbegriff ist "Local Area Network", kurz "LAN") oder um ein großräumiges Netzwerk (ein englischer Fachbegriff ist "Wide Area Network", kurz "WAN") handeln. Ein Beispiel für ein lokales Netzwerk ist ein Intranet, ein Beispiel für ein großräumiges Netzwerk ist das Internet. Das Netzwerk NETW kann insbesondere auch drahtlos ausgeführt sein, insbesondere als WLAN (für "wireless LAN", im englischen ist die Abkürzung "WiFi" gebräuchlich) oder als Bluetooth-Verbindung. Das Netzwerk NETW kann auch als Kombination der genannten Beispiele ausgeführt sein.

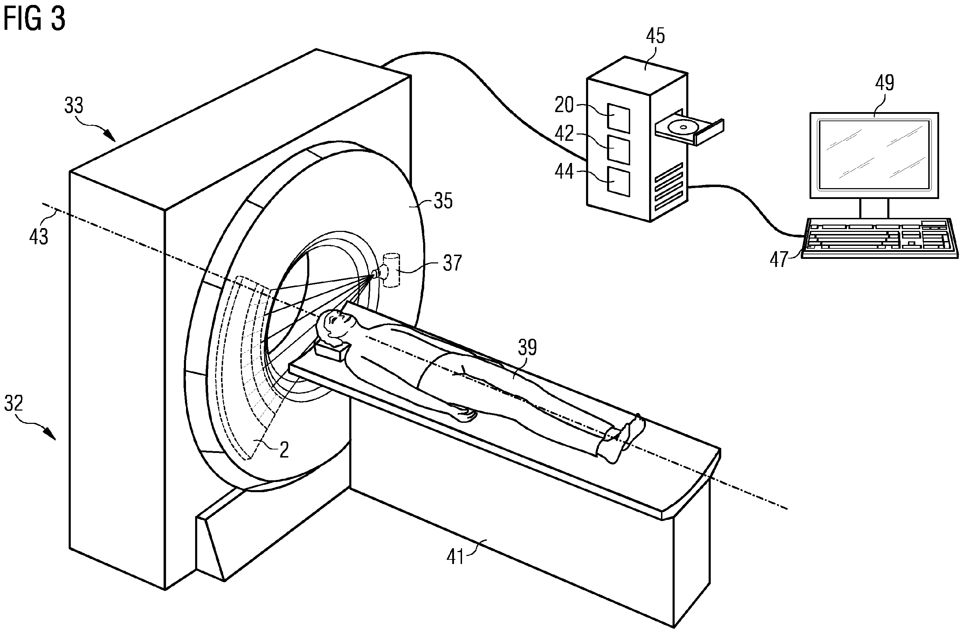

Fig. 3 zeigt eine beispielhafte Ausführungsform eines Röntgenbildgebungssystem 32 mit einem Röntgendetektor 2 und einer Röntgenquelle 37 in Gegenüberstellung zum Röntgendetektor 2. Die Röntgenquelle 37 ist ausgebildet, den Röntgendetektor 2 mit Röntgenstrahlung zu belichten.

Das gezeigte Röntgenbildgebungssystem 32 ist insbesondere als Computertomographie-Gerät ausgebildet. Ein erfindungsgemäßes Röntgenbildgebungssystem kann in anderen Ausbildungsvarianten auch beispielsweise als SPECT- oder PET-System, als C-Bogen-Röntgengerät oder Dyna-CT ausgebildet sein. Ebenso könnte es als Mammographie-Gerät oder als Radiographie-Gerät ausgebildet sein

Das Computertomographie-Gerät umfasst eine Gantry 33 mit einem Rotor 35. Der Rotor 35 umfasst die Röntgenquelle 37 und den Röntgendetektor 2. Der Rotor 35 ist um die Rotationsachse 43 drehbar. Das Untersuchungsobjekt 39, hier ein Patient, ist auf der Patientenliege 41 gelagert und ist entlang der Rotationsachse 43 durch die Gantry 33 bewegbar. Im Allgemeinen kann das Objekt 39 beispielsweise einen tierischen Patienten und/oder einen menschlichen Patienten umfassen.

Zur Steuerung des Röntgenbildgebungssystem 32 und/oder zur Erzeugung eines Röntgenbilddatensatzes basierend auf von dem Röntgendetektor 2 verarbeiteten elektrischen Signalen basierend auf der eingestrahlten Röntgenstrahlung ist eine Verarbeitungseinheit 45 umfassend eine Steuereinheit 42 und eine Rekonstruktionseinheit 44 vorgesehen. Die Verarbeitungseinheit 45 umfasst außerdem eine Vorrichtung 20 zur Ermittlung eines korrigierten Röntgenbilddatensatzes.

Im Falle eines Computertomographie-Geräts wird üblicherweise aus einer Vielzahl an Winkelrichtungen ein (Roh-) Röntgenbilddatensatz des Objekts mittels der Detektionseinheit 2 aufgenommen, welcher auf den verarbeiteten elektrischen Signalen des Röntgendetektors 2 basiert, während der Patient 39 kontinuierlich oder sequenziell durch die Gantry 33 mittels der Patientenliege 41 bewegt wird. Anschließend kann basierend auf dem (Roh-) Röntgenbilddatensatz mittels eines mathematischen Verfahrens, beispielsweise umfassend eine gefilterte Rückprojektion oder ein iteratives Rekonstruktionsverfahren, ein finaler, beispielsweise dreidimensionaler, Röntgenbilddatensatz oder eine Mehrzahl an Schichtbilddatensätzen rekonstruiert werden.

Der Röntgendetektor 2 ist insbesondere als photonen-zählender Röntgendetektor ausgebildet, welcher ausgebildet ist Messdaten in Abhängigkeit von zumindest zwei Röntgenenergieschwellwerten aufzunehmen, auf welchen basierend ein vorläufiger spektraler Röntgenbilddatensatz darstellend eine ortsaufgelöste Abschwächung von Röntgenstrahlung beim Durchgang durch das Objekt 39 und umfassend zumindest erste Bildwerte in Abhängigkeit eines ersten Röntgenenergieschwellwerts und zweite Bildwerte in Abhängigkeit eines zweiten Röntgenenergieschwellwerts bereitgestellt werden kann.

Dem Patient 39 kann mit Hilfe eines Kontrastmittelapplikators ein Kontrastmittelbolus injiziert werden, so dass ein kontrastverstärkter Röntgenbilddatensatz erzeugt werden kann.

Des Weiteren ist eine Eingabeeinrichtung 47 und eine Ausgabeeinrichtung 49 mit der Recheneinheit 45 verbunden. Die Eingabeeinrichtung und die Ausgabeeinrichtung können beispielsweise eine Interaktion, beispielsweise eine manuelle Konfiguration des Röntgenbildgebungssystems 32, eine Bestätigung oder ein Auslösen eines Verfahrensschritts durch einen Anwender ermöglichen.

**Patentansprüche**

1. Verfahren zur Ermittlung eines korrigierten Röntgenbilddatensatzes umfassend die Schritte

   - Bereitstellen (S1) eines vorläufigen spektralen Röntgenbilddatensatzes darstellend eine ortsaufgelöste Abschwächung von Röntgenstrahlung beim Durchgang durch ein Objekt (39) umfassend zumindest erste Bildwerte in Abhängigkeit eines ersten Röntgenenergieschwellwerts und zweite Bildwerte in Abhängigkeit eines zweiten Röntgenenergieschwellwerts,
   - Korrigieren (S2) zumindest eines der ersten Bildwerte oder eines der zweiten Bildwerte des vorläufigen Röntgenbilddatensatzes mittels einer Skalierungsfunktion in Abhängigkeit eines Röntgenphotonenflusses und einer Kombination aus dem zu korrigierenden Bildwert der ersten oder zweiten Bildwerte und dem dazu örtlich korrespondierendem Bildwert der jeweils dem anderen Röntgenenergieschwellwert zugeordneten Bildwerte,
   - Ausgeben (S3) des korrigierten Röntgenbilddatensatzes umfassend den zumindest einen korrigierten Bildwert der ersten oder zweiten Bildwerte.

2. Verfahren nach Anspruch 1, wobei die Kombination aus sowohl dem zu korrigierenden Bildwert als auch dem dazu örtlich korrespondierendem Bildwert der jeweils dem anderen Röntgenenergieschwellwert zugeordneten Bildwerte einer Differenz der jeweiligen Bildwerte oder einem Quotienten aus den jeweiligen Bildwerten entspricht.

3. Verfahren nach einem der Ansprüche 1 oder 2, wobei sowohl ein Bildwert der ersten Bildwerte als auch ein Bildwert der zweiten Bildwerte korrigiert wird, wobei sich zumindest ein Parameter der Skalierungsfunktion für den zu korrigierenden Bildwert der ersten Bildwerte und für den zu korrigierenden Bildwert der zweiten Bildwerte unterscheidet.

4. Verfahren nach einem der vorangehenden Ansprüche, wobei die Skalierungsfunktion außerdem abhängig ist von zumindest einem der folgenden Größen:

   - einem Einstellungsparameter einer zur Aufnahme des spektralen Röntgenbilddatensatzes eingesetzten Röntgenquelle,
   - einem Formfilterparameter eines zur Aufnahme des spektralen Röntgenbilddatensatzes eingesetzten Formfilters,
   - einem Filterparameter eines zur Aufnahme des spektralen Röntgenbilddatensatzes eingesetzten spektralen Filters, oder
   - dem ersten oder zweiten Röntgenenergieschwellwert.

5. Verfahren nach einem der vorangehenden Ansprüche, wobei im Schritt des Korrigierens (S2) der zumindest eine Bildwert der ersten Bildwerte oder der zweiten Bildwerte erst ab einem minimalen Röntgenphotonenfluss mittels der Skalierungsfunktion korrigiert wird.

6. Verfahren nach einem der vorangehenden Ansprüche, wobei die Skalierungsfunktion eine Exponentialfunktion oder ein Polynom höherer Ordnung umfasst.

7. Verfahren nach einem der vorangehenden Ansprüche, wobei der Röntgenbilddatensatz ein Computertomographie-Bilddatensatz, insbesondere ein Projektionsdatensatz, ist.

8. Verfahren nach Anspruch 7, wobei die Korrektur zumindest des einen der ersten Bildwerte oder des einen der zweiten Bildwerte nach einer Wasserskalierung der ersten oder zweiten Bildwerte durchgeführt wird.

9. Verfahren nach einem der vorangehenden Ansprüche, wobei der Röntgenbilddatensatz ein kontrastverstärkter Röntgenbilddatensatz ist.

10. Verfahren nach einem der vorangehenden Ansprüche, wobei eine Mehrzahl an spektralen Röntgenbilddatenätzen unter Verwendung unterschiedlicher Röntgenphotonenflüsse bereitgestellt werden und für jeden der bereitgestellten spektralen Röntgenbilddatensätze jeweils der Schritt des Korrigierens durchgeführt wird.

11. Vorrichtung (20) zur Ermittlung eines korrigierten Röntgenbilddatensatzes eines Objekts (39) umfassend

   - eine erste Schnittstelle (21) ausgebildet zum Bereitstellen eines vorläufigen spektralen Röntgenbilddatensatzes umfassend zumindest erste Bildwerte in Abhängigkeit eines ersten Röntgenenergieschwellwerts und zweite Bildwerte in Abhängigkeit eines zweiten Röntgenenergieschwellwerts,
   - eine Recheneinheit (23) ausgebildet zum Korrigieren zumindest eines der ersten Bildwerte oder eines der zweiten Bildwerte des vorläufigen Röntgenbilddatensatzes mittels einer Skalierungsfunktion in Abhängigkeit eines Röntgenphotonenflusses und einer Kombination aus sowohl dem zu korrigierenden Bildwert als auch dem dazu örtlich korrespondierendem Bildwert der jeweils dem anderen Röntgenenergieschwellwert zugeordneten Bildwerte, und
   - eine zweite Schnittstelle (25) ausgebildet zum Ausgeben des korrigierten Röntgenbilddatensatzes umfassend den zumindest einen korrigierten Bildwert der ersten oder zweiten Bildwerte.

12. Röntgenbildgebungssystem (32) umfassend eine Vorrichtung nach Anspruch 11 und eine Röntgenquelle (37) in Gegenüberstellung zu einem Röntgendetektor (2), wobei zwischen die Röntgenquelle (37) und den Röntgendetektor (2) zur Aufnahme des spektralen Röntgenbilddatensatz ein Objekt (39) platzierbar ist.

13. Röntgenbildgebungssystem (32) nach Anspruch 12, wobei das Röntgenbildgebungssystem ein Computertomographie-Gerät ist.

14. Computerprogrammprodukt mit einem Computerprogramm, welches direkt in einen Speicher (27) einer Vorrichtung (20) zur Ermittlung eines korrigierten Röntgenbilddatensatzes ladbar ist, mit Programmabschnitten, um alle Schritte des Verfahrens nach einem der Ansprüche 1 bis 10 auszuführen, wenn die Programmabschnitte von der Vorrichtung (20) ausgeführt werden.

15. Computerlesbares Speichermedium, auf welchem von einer Vorrichtung (20) zur Ermittlung eines korrigierten Röntgenbilddatensatzes lesbare und ausführbare Programmabschnitte gespeichert sind, um alle Schritte des Verfahrens nach einem der Ansprüche 1 bis 10 auszuführen, wenn die Programmabschnitte von der Vorrichtung (20) ausgeführt werden.

## FIG 1

S12

S1

S2

S3

S13

## FIG 2

21

23

25

27

20

32

FIG 3

Europäisches
Patentamt

European
Patent Office

Office européen
des brevets

# EUROPÄISCHER RECHERCHENBERICHT

Nummer der Anmeldung

EP 21 18 2696

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CHRISTENSEN ERIK D ET AL: "Spectral correction algorithm for multispectral CdTe x-ray detectors", PROCEEDINGS OF SPIE; [PROCEEDINGS OF SPIE ISSN 0277-786X VOLUME 10524], SPIE, US, Bd. 10393, 7. September 2017 (2017-09-07), Seiten 103930H-103930H, XP060097065, DOI: 10.1117/12.2272935 ISBN: 978-1-5106-1533-5 | 1,3-6, 9-11,14, 15 | INV. G06T5/50 A61B6/00 |
| Y | * Seite 1, rechte Spalte, letzter Absatz * * Seite 2, linke Spalte, Zeilen 9-18 * * Gleichungen 19 und 20 * * Kapitel "4.2 Optimizing the Flux-Dependent Coefficients" * * Seite 7, rechte Spalte, Zeilen 5-7 * * Abbildung 9 * ----- | 8 | |
| X | HSIEH SCOTT S: "Coincidence Counters for Charge Sharing Compensation in Spectroscopic Photon Counting Detectors", IEEE TRANSACTIONS ON MEDICAL IMAGING, IEEE, USA, Bd. 39, Nr. 3, 8. August 2019 (2019-08-08) , Seiten 678-687, XP011775598, ISSN: 0278-0062, DOI: 10.1109/TMI.2019.2933986 [gefunden am 2020-03-02] | 1-4,7, 9-15 | |
| Y | * Titel * * Abbildungen 1,3 * * Seite 686, linke Spalte, Zeilen 32-35 * * Zusammenfassung * * Seite 687, linke Spalte, Zeile 3 * ----- -/-- | 8 | RECHERCHIERTE SACHGEBIETE (IPC) G06T A61B |

Der vorliegende Recherchenbericht wurde für alle Patentansprüche erstellt

| Recherchenort | Abschlußdatum der Recherche | Prüfer |
|---|---|---|
| München | 5. Dezember 2021 | Winkler, Gregor |

EPO FORM 1503 03.82 (P04C03)

Seite 1 von 2

Europäisches
Patentamt

European
Patent Office

Office européen
des brevets

# EUROPÄISCHER RECHERCHENBERICHT

Nummer der Anmeldung

EP 21 18 2696

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | KAPPLER S ET AL: "Photon counting CT at elevated X-ray tube currents: contrast stability, image noise and multi-energy performance", PROGRESS IN BIOMEDICAL OPTICS AND IMAGING, SPIE - INTERNATIONAL SOCIETY FOR OPTICAL ENGINEERING, BELLINGHAM, WA, US, Bd. 9033, 19. März 2014 (2014-03-19), Seiten 90331C-90331C, XP060031513, ISSN: 1605-7422, DOI: 10.1117/12.2043511 ISBN: 978-1-5106-0027-0 * Seite 2, vorletzter Absatz, Zeilen 1-4 * ----- | 8 | |
| | | | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| München | 5. Dezember 2021 | Winkler, Gregor |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
...................................................................
& : Mitglied der gleichen Patentfamilie, übereinstimmendes
Dokument

EPO FORM 1503 03.82 (P04C03)

Seite 2 von 2